# FASCICULE DE BREVET EUROPEEN

(11) **EP 2 611 404 B1**
(45) Date de publication et mention de la délivrance du brevet: **30.07.2014**
(21) Numéro de dépôt: 11743108.0
(22) Date de dépôt: 12.07.2011
(51) Int. Cl.: D04B 1/26

(54) **ORTHESE DE COMPRESSION**
KOMPRESSIONSORTHESE
COMPRESSION ORTHOSIS

(30) Priorité: 30.08.2010 FR 1056837
(43) Date de publication de la demande: 10.07.2013
(73) Titulaire: Gibaud, 42000 Saint Etienne (FR)
(72) Inventeur: VALOIS, Christophe, F-01090 Montmerle (FR); MCGOWAN, Michael, F-38330 Montbonnot Saint Martin (FR)
(74) Mandataire: Delorme, Nicolas
(86) Numéro de dépôt international: PCT/FR2011/051656
(87) Numéro de publication internationale: WO 2012/028795

(56) Documents cités:
- DE-U1- 8 606 402
- FR-A1- 2 445 701
- US-A- 3 162 029
- US-A- 4 149 274
- US-A- 4 282 726
- US-A- 4 702 091

## Description

La présente invention concerne une orthèse de compression notamment un bas, collant ou chaussette de contention.

Le port de ces orthèses est prescrit en cas d'insuffisance veineuse et plus particulièrement en cas d'insuffisance veineuse superficielle.

Dans ces cas, le flux sanguin ne se fait plus dans des conditions satisfaisantes ; le sang stagne et les veines superficielles se dilatent.

Outre des traitements médicamenteux phlébologiques, l'insuffisance veineuse peut être traitée de manière orthopédique par des collants, bas ou chaussettes de compression qui appliquent une pression dégressive sur le membre inférieure. Les orthèses de compression qui sont fortement élastiques permettent de faire remonter le sang vers le coeur par une pression plus forte à la cheville qu'au niveau du mollet ou de la cuisse, comme par exemple celles décrites dans les documents DE 806402 U1, US 4149274, FR 2445701, US 4282726 US 3162029.

Par nécessité fonctionnelle, les orthèses de contention sont réalisées dans une matière textile fortement élastique et il convient d'en améliorer le confort. Le port d'une orthèse de contention sera en effet d'autant mieux observé que l'orthèse est d'un porté confortable.

Dans ce contexte, le but de l'invention est de proposer une orthèse de compression qui présente un confort amélioré sans pour autant dégrader ses propriétés de compression ni renchérir son coût de fabrication.

L'invention concerne une orthèse de compression d'un membre inférieur constitué d'un tricot de compression caractérisée ce que l'orthèse de compression comprend au moins une zone gaufrée définie par un tricot présentant :
- un premier type de mailles liant des fils élastiques de tricotage, des fils non élastique de tricotage et des fils élastique de trame, et
- un second type de mailles dont le liage est défini par le fait que :
   - les fils élastiques de tricotage présentent des mailles allongées sur au moins deux rangées et que
   - les fils élastiques de trame présentent des flottés de, au moins, deux aiguilles et que
   - les fils non élastiques de tricotage présentent des flottés de, au moins une aiguille.

L'invention propose ainsi un liage original dans une zone particulière d'une orthèse de manière à créer un gaufrage dont l'épaisseur va procurer un surcroit de confort. Il est à noter que le gaufrage fait partie intégrante du tricot qui forme l'orthèse.

Dans une forme de réalisation préférée, dans la zone de gaufrage les fils élastiques de tricotage sont allongés sur six rangées.

Dans une forme de réalisation préférée, dans la zone de gaufrage, les fils élastiques de trame présentent des flottés de trois aiguilles.

Dans une forme de réalisation préférée, dans la zone de gaufrage, les fils non élastiques de tricotage présentent des flottés d'une aiguille.

Par ailleurs, le premier type de maille peut être un jersey tramé. Selon des dispositions préférées de l'invention :
- le fil élastique de tricotage est un élasthanne guipé ;
- le fil non élastique de tricot est un polyamide ;
- le fil élastique de trame est un élasthanne double guipé.

Dans une application particulièrement avantageuse de l'invention, la zone de gaufrage est positionnée au niveau de la semelle d'une orthèse du groupe comprenant notamment une chaussette, un mi-bas, un bas, un collant.

Pour sa bonne compréhension, l'invention est décrite en référence au dessin ci annexé montrant une forme de réalisation d'une chaussette de contention selon celle-ci.
Figure 1 représente schématiquement une chaussette selon l'invention ;
Figures 2 et 3 montrent la partie semelle de cette chaussette en vue de dessous et en vue de coté ;
Figure 4 représente un diagramme de liage de la partie semelle de la chaussette des figures 1 à 3.

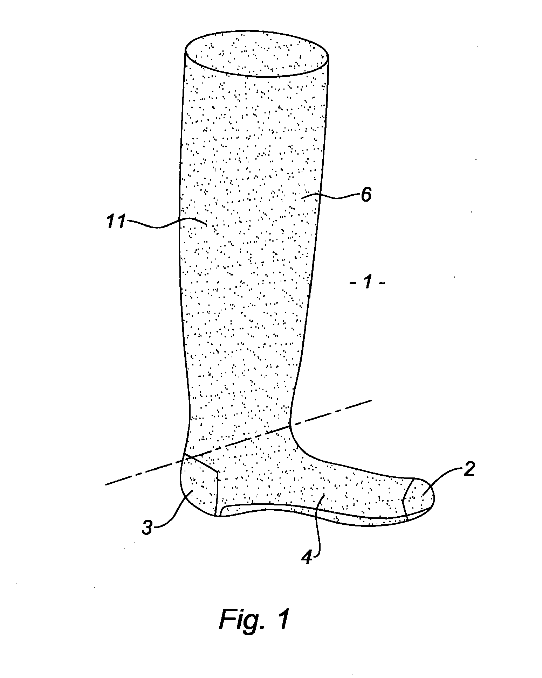

La figure 1 montre de façon globale une chaussette qui porte la référence 1. Il s'agit d'une orthèse de compression tubulaire en textile tricoté. Les fils intervenants dans le tricot et la structure de maille sont déterminés pour que la chaussette exerce une contention dégressive sur le membre une fois que la chaussette est en place sur celui-ci.

Le chaussette 1 représentée à la figure 1 est du type mi bas et comprend ainsi une partie qui enveloppe le pied et une partie qui enveloppe la cheville et le mollet.

De façon connue en soi, cette chaussette 1 est conçue pour produire une pression de 10 mm de Hg (minimum pour une orthèse référencée en classe 1) à 36 mm de Hg (minimum pour une orthèse référencée en classe 4) - le mm de Hg étant l'unité usuelle en phlébologie - mesurée au niveau de la cheville.

La chaussette 1 présente un tricot de compression conventionnel qui lui permet d'offrir la contention requise

Il est tout à fait possible de conférer à la chaussette 1 un aspect lisse, côtelé ou éventuellement chiné.

Comme cela est également habituel, un traitement particulier peut être réservé aux extrémités avant 2 et arrière 3 du pied.

Sur les parties de la chaussette qui enveloppent la cheville et le talon et sur la partie supérieure du pied, le tricotage est conventionnel et peut être constitué de mailles jersey tramées.

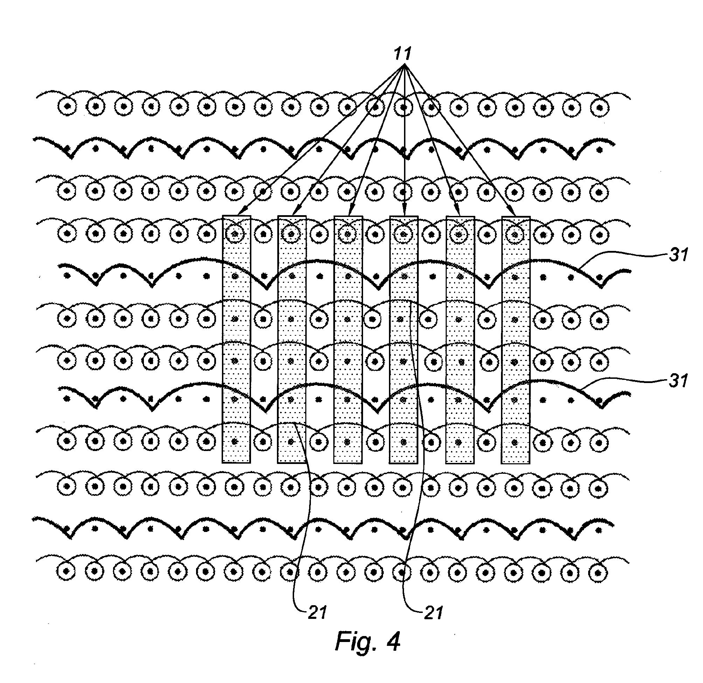

Elle est également dotée d'un tricot original qui est représenté sur la figure 4. Le tricot inclut :
- un premier fil élastique de tricotage 10 par exemple en élasthanne guipé polyamide et/ou coton ;
- un deuxième fil non élastique de tricotage 20 par exemple en polyamide ;
- un fil de trame élastique 30 qui produit la pression élastique par exemple, en élasthanne ou à base d'un mélange d'élasthanne guipé (par exemple polyamide).

Un aspect important de l'invention est le traitement de la partie semelle 4 de la chaussette 1 c'est-à-dire la partie de la chaussette 1 qui est en regard de la plante du pied.

La partie de semelle 4 de la chaussette 1 n'est pas une partie rapportée mais est réalisée au cours de la séquence de tricotage de la chaussette 1.

Il est ainsi prévu une modification substantielle de la maille qui forme la partie semelle 4 de la chaussette 1.

La contexture de maille de la partie semelle 4 de la chaussette 1 est tout à fait originale.

Dans cette partie de la chaussette 1, on note deux dispositions originales combinées qui apparaissent sur le diagramme de liage de la figure 4.

D'une part, les fils de tricotage élastique 10 sont tricotés selon des mailles allongées 11 sur plusieurs rangs. En pratique les mailles peuvent s'allonger sur six rangs. Les mailles allongées 11 apparaissent par convention sur le diagramme de liage en grisé. De manière concrète, ces mailles allongées 11 sont réalisées en mettant en attente des aiguilles qui ont chargé. Ainsi, les mailles restées en attente sur les aiguilles qui ont chargé, s'allongent pour former des fils flottants. Les mailles allongées apparaissent sur l'endroit de la semelle 4.

D'autre part, les fils de trame 30 présentent des flottés 31 de trois à l'intersection avec les mailles allongées. Hors de la zone de mailles allongées 11, les fils de trame 30 présentent un flotté d'une aiguille.

De plus, les fils de tricot non élastiques 20 présentent des flottés 21 comme on peut le voir sur la figure 4 de une aiguille à l'intersection avec les mailles allongées. Hors de la zone de mailles allongées 11, les fils de tricot non élastiques 20 présentent un jersey toutes aiguilles.

L'effet combiné des mailles allongées 11 des fils de tricotage élastiques 30 et des flottés 31 des fils élastiques de trame 10 et des flottés 21 des fils non élastiques 20 a pour effet remarquable de créer un effet de gaufrage sur la semelle de la chaussette 1.

En effet lors la réalisation de la chaussette 1, le fil de tricotage élastique 10, le fil de tricot non élastiques 20 et le fil de trame élastique 30 sont maintenus sous tension dans la machine à tricoter.

En revanche, à la tombée de la machine, les fils élastiques de tricotage 10, les fils de tricot non élastiques 20 et les fils élastiques de trame 30 ne sont plus maintenus en extension et prennent leur état normal contracté en créant des creux et des reliefs.

Dans l'exemple représenté, le gaufrage est présent dans la semelle au niveau du talon et du métatarse qui sont les points d'appui principaux du corps.

Ce gaufrage confère une surépaisseur à la semelle de la chaussette 1 qui s'avère extrêmement confortable au porté et a ainsi un effet bénéfique sur l'observance du port de la chaussette 1 de contention. Le gaufrage absorbe en effet une partie du poids du corps.

Le gaufrage apparaît distinctement sur la face « endroit » de la semelle sur laquelle un réseau de reliefs et de creux de contours carrés essentiellement générés par les mailles allongées des fils de tricotage élastiques tandis que la face envers de la semelle fait apparaître un série de bourrelets parallèles essentiellement générés par les flottées des fils de trame élastiques 30.

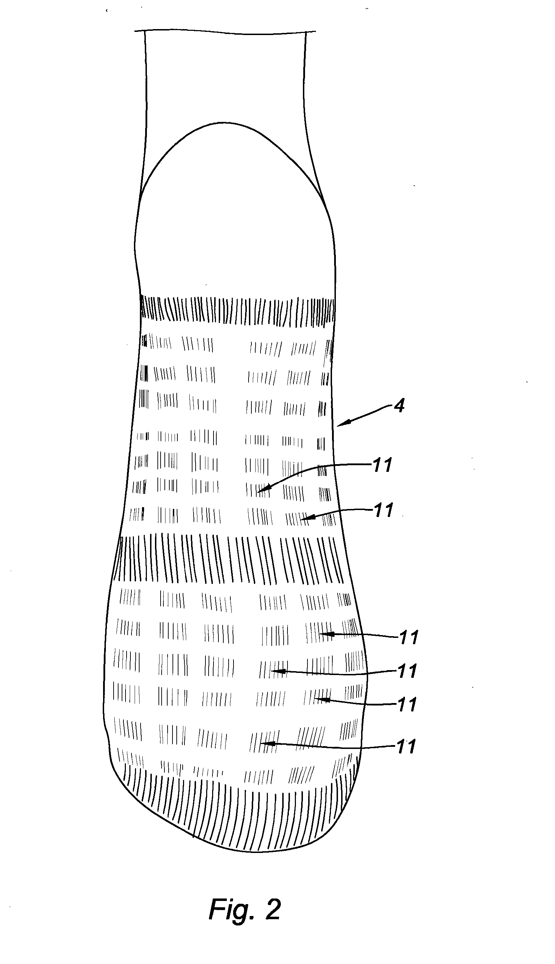

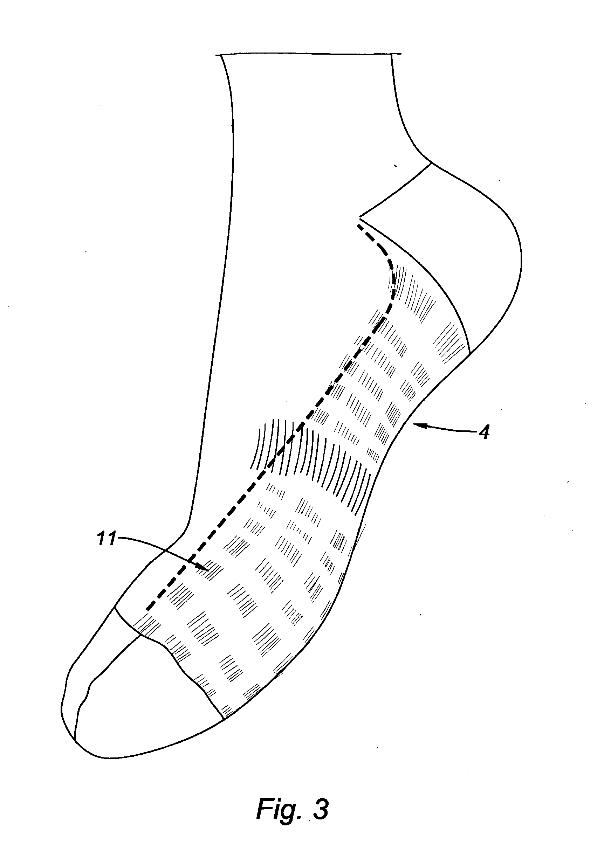

Comme on peut le voir sur la figure 2 ou sur la figure 3, des séries de six mailles allongées forment des saillies qui alternent transversalement avec des séries de six mailles jersey qui forment des creux.

Un point tout à fait intéressant à mentionner est que la zone gaufrée de semelle 4 qui est spécifique de l'invention peut être incorporée à tout type d'orthèse de contention. En d'autres termes, la zone de gaufrage est compatible avec tout type de compression veineuse élastique. Ainsi, il peut être envisagé de créer une zone gaufrée dans des orthèses appartenant aux classes usuelles I, II, III ou IV.

Un autre point qu'il convient de souligner est que la zone de gaufrage peut être positionnée à d'autres zones que la zone de semelle d'une orthèse. Ainsi, la zone de gaufrage pourrait être située au niveau d'une articulation pour apporter un surcroit de confort dans une zone particulière.

Bien entendu, l'invention n'est pas limitée à la forme de réalisation décrite ci-dessus à titre d'exemple non limitatif mais elle en embrasse au contraire toutes les formes de réalisation.

## Revendications

1. Orthèse de compression (1) d'un membre inférieur constitué d'un tricot de compression caractérisée ce que l'orthèse de compression comprend au moins une zone gaufrée définie par un tricot présentant :
- un premier type de mailles liant des fils élastiques de tricotage (10), des fils non élastique de tricotage (20) et des fils élastique de trame (30), et
- un second type de mailles dont le liage est défini par le fait que :
- les fils élastiques de tricotage (10) présentent des mailles allongées (11) sur au moins deux rangées et que
- les fils élastiques de trame (30) présentent des flottés (31) de, au moins, deux aiguilles et que
- les fils non élastiques de tricotage (20) présentent des flottés (21) de, au moins une aiguille.

2. Orthèse de compression selon la revendication 1, **caractérisée en ce que**, dans la zone de gaufrage les fils élastiques de tricotage (10) sont allongés sur six rangées.

3. Orthèse de compression selon la revendication 1 ou la revendication 2, **caractérisée en ce que**, dans la zone de gaufrage, les fils élastiques de trame (30) présentent des flottés (31) de trois aiguilles.

4. Orthèse de compression selon la revendication 1 ou la revendication 2, ou la revendication 3, **caractérisée en ce que**, dans la zone de gaufrage, les fils non élastiques de tricot (20) présentent des flottés (21) de une aiguille.

5. Orthèse de compression selon l'une des revendications 1 à 4, **caractérisée en ce que** le premier type de maille est un jersey tramé dit toutes aiguilles.

6. Orthèse de compression selon l'une des revendications 1 à 5, **caractérisée en ce que** le fil élastique de tricotage (10) est un élasthanne guipé.

7. Orthèse de compression selon l'une des revendications 1 à 6, **caractérisé en ce que** le fil non élastique de tricot (20) est un polyamide.

8. Orthèse de compression selon l'une des revendications 1 à 7, **caractérisée en ce que** le fil élastique de trame (30) est un élasthanne double guipé.

9. Orthèse de compression selon l'une des revendications 1 à 8, **caractérisée en ce que** la zone de gaufrage est positionnée au niveau de la semelle (4) d'une orthèse du groupe comprenant notamment une chaussette, un mi-bas, un bas, un collant.

## Patentansprüche

1. Kompressionsorthese (1) einer unteren Extremität, umfassend ein Kompressionsgestrick, **dadurch gekennzeichnet, dass** die Kompressionsorhtese mindestens einen geprägten Teil umfasst, der durch ein Gestrick definiert ist, der Folgendes umfasst:
- eine erste Art von Maschen, die elastische Strickgarne (10), nicht elastische Strickgarne (20) und elastische Schussgarne (30) verbinden, und
- eine zweite Art von Maschen, deren Verbindung durch die Tatsache definiert ist, dass:
- die elastischen Strickgarne (10) verlängerte Maschen (11) auf mindestens zwei Reihen aufweisen, und dass
- die elastischen Schussgarne (30) Flottierungen (31) von mindestens zwei Nadeln aufweisen, und dass
- die nicht elastischen Strickgarne (20) Flottierungen (21) von mindestens einer Nadel aufweisen.

2. Kompressionsorthese nach Anspruch 1, **dadurch gekennzeichnet, dass** im Bereich der Prägung die elastischen Strickgarne (10) über sechs Reihen verlängert sind.

3. Kompressionsorthese nach Anspruch 1 oder Anspruch 2, **dadurch gekennzeichnet, dass** im Bereich der Prägung die elastischen Schussgarne (30) Flottierungen (31) von drei Nadeln aufweisen.

4. Kompressionsorthese nach Anspruch 1 oder Anspruch 2 oder Anspruch 3, **dadurch gekennzeichnet, dass** im Bereich der Prägung die nicht elastischen Strickgarne (20) Flottierungen (21) von einer Nadel aufweisen.

5. Kompressionsorthese nach einem der Ansprüche 1 bis 4, **dadurch gekennzeichnet, dass** die erste Art von Maschen ein geschossener Jersey, genannt von allen Nadeln, ist.

6. Kompressionsorthese nach einem der Ansprüche 1 bis 5, **dadurch gekennzeichnet, dass** das elastische Strickgarn (10) ein umsponnenes Elasthan ist.

7. Kompressionsorthese nach einem der Ansprüche 1 bis 6, **dadurch gekennzeichnet, dass** das nicht elastische Strickgarn (20) ein Polyamid ist.

8. Kompressionsorthese nach einem der Ansprüche 1 bis 7, **dadurch gekennzeichnet, dass** das elastische Schussgarn (30) ein doppelt umsponnenes Elasthan ist.

9. Kompressionsorthese nach einem der Ansprüche 1 bis 8, **dadurch gekennzeichnet, dass** der Bereich der Prägung auf der Ebene der Sohle (4) einer Orthese der Gruppe, umfassend insbesondere eine Socke, einen Halbstrumpf, einen Strumpfe, eine Strumpfhose, positioniert ist.

## Claims

1. A compression orthosis (1) of a lower limb consisting of a compression knit **characterized in that** the compression orthosis comprises at least one embossed area defined by a knit having:
- A first type of stitches binding elastic knitting yarns (10), non-elastic knitting yarns (20) and elastic weft yarns (30), and
- A second type of stitches, binding of which is defined by:
- The elastic knitting yarns (10) having elongated stitches (11) on at least two rows and by
- The elastic weft yarns (30) having floats (31) of, at least, two needles and by
- The non-elastic knitting yarns (20) having floats (21) of at least one needle.

2. The compression orthosis according to claim 1, **characterized in that**, in the embossing area, the elastic knitting yarns (10) lie on six rows.

3. The compression orthosis according to claim 1 or claim 2, **characterized in that**, in the embossing area, the elastic weft yarns (30) have floats (31) of three needles.

4. The compression orthosis according to claim 1 or claim 2, or claim 3, **characterized in that**, in the embossing area, the non-elastic knitting yarns (20) have floats (21) of one needle.

5. The compression orthosis according to any of claims 1 to 4, **characterized in that** the first type of stitch is a weft jersey called all needles.

6. The compression orthosis according to any of claims 1 to 5, **characterized in that** the elastic knitting yarn (10) is a covered elastane.

7. The compression orthosis according to any of claims 1 to 6, **characterized in that** the non-elastic knitting yarn (20) is a polyamide.

8. The compression orthosis according to any of claims 1 to 7, **characterized in that** the elastic weft yarn (30) is a double covered elastane.

9. The compression orthosis according to any of claims 1 to 8, **characterized in that** the embossing area is positioned at the sole (4) of an orthosis of the group comprising in particular a sock, a knee-high sock, a stocking, a tight.
